(19) Europäisches Patentamt
European Patent Office
Office européen des brevets

(11) Veröffentlichungsnummer: **0 180 818**
**A2**

(12) # EUROPÄISCHE PATENTANMELDUNG

(21) Anmeldenummer: 85113059.1

(22) Anmeldetag: 15.10.85

(51) Int. Cl.⁴: C 07 C 127/22
C 07 C 93/14, C 07 C 79/35
C 07 C 121/75, C 07 C 65/21
C 07 C 119/048, C 07 C 161/04
A 01 N 47/34

(30) Priorität: 27.10.84 DE 3439364

(43) Veröffentlichungstag der Anmeldung:
14.05.86 Patentblatt 86/20

(84) Benannte Vertragsstaaten:
AT BE CH DE FR GB IT LI NL

(71) Anmelder: BAYER AG
Konzernverwaltung RP Patentabteilung
D-5090 Leverkusen 1 Bayerwerk(DE)

(72) Erfinder: Sirrenberg, Wilhelm, Dr.
Wuppertaler Strasse 21
D-4322 Sprockhövel(DE)

(72) Erfinder: Marhold, Albrecht, Dr.
Carl-Duisberg-Strasse 329
D-5090 Leverkusen 1(DE)

(72) Erfinder: Becker, Benedikt, Dr.
Metzkausener Strasse 14
D-4020 Mettmann(DE)

(72) Erfinder: Stendel, Wilhelm, Dr.
In den Birken 55
D-5600 Wuppertal 1(DE)

(54) **Benzoylharnstoffe und Zwischenprodukte.**

(57) Die vorliegende Erfindung betrifft neue 1-Phenyl-3-benzoyl-(thio)harnstoffe der allgemeinen Formel (I)

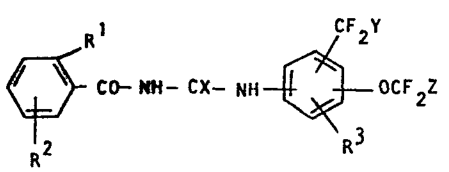

(I)

gefunden,
in welcher
$R^1$ für Halogen oder Alkyl steht,
$R^2$ für Wasserstoff, Halogen oder Alkyl,
$R^3$ für Wasserstoff, Fluor oder Chlor steht,
X für Sauerstoff oder Schwefel steht und
Y und Z gleich oder verschieden sind und für Fluor oder Chlor stehen,
welche als Schädlingsbekämpfungsmittel verwendet werden können, sowie Zwischenprodukte zur Herstellung dieser Verbindungen.

EP 0 180 818 A2

BAYER AKTIENGESELLSCHAFT 5090 Leverkusen, Bayerwerk
Konzernverwaltung RP
Patentabteilung S/ABc

## Benzoylharnstoffe und Zwischenprodukte

Die vorliegende Erfindung betrifft neue substituierte 1-Phenyl-3-benzoyl-(thio)harnstoffe, mehrere Verfahren zu ihrer Herstellung und ihre Verwendung als Schädlingsgekämpfungsmittel, insbesondere als Insektizide und Akarizide sowie Zwischenprodukte zu ihrer Herstellung.

Es ist bereits bekannt, daß bestimmte Benzoylharnstoffe insektizide Eigenschaften besitzen (vgl. z.B. US-PS 4 139 636, US-PS 3 748 356, EP-A-3099 und EP-A-23 884).

Es wurden die neuen substituierten 1-Phenyl-3-benzoyl-(thio)harnstoffe der allgemeinen Formel (I)

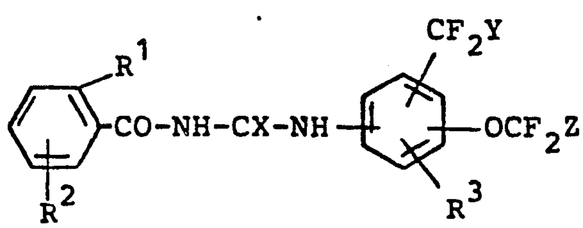

(I)

gefunden,

in welcher

$R^1$ für Halogen oder Alkyl steht,

$R^2$ für Wasserstoff, Halogen oder Alkyl,

$R^3$ für Wasserstoff, Fluor oder Chlor steht,

X für Sauerstoff oder Schwefel steht und

Y und Z gleich oder verschieden sind und für Fluor oder Chlor stehen.

Diese neuen Verbindungen weisen starke biologische, insbesondere insektizide Eigenschaften auf, die ihre Verwendung als Schädlingsbekämpfungsmittel, insbesondere als Insektizide und Akarizide ermöglichen.

Weiterhin wurde gefunden, daß die neuen substituierten 1-Phenyl-3-benzoyl-(thio)harnstoffe der allgemeinen Formel (I) erhalten werden, indem man

a) substituierte Aniline der allgemeinen Formel (II)

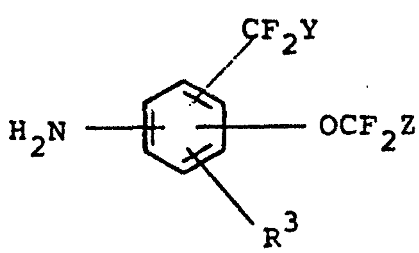

(II)

in welcher

$R^3$, Y und Z die oben angegebene Bedeutung haben,

mit Benzoyl-iso(thio)cyanaten der allgemeinen Formel (III)

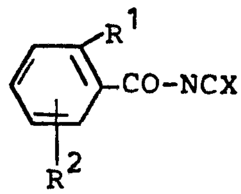

(III)

in welcher

X, $R^1$ und $R^2$ die oben angegebene Bedeutung haben,

gegebenenfalls in Gegenwart eines Verdünnungsmittels umsetzt oder

b) substituierte Phenyl-iso(thio)cyanate der allgemeinen Formel (IV)

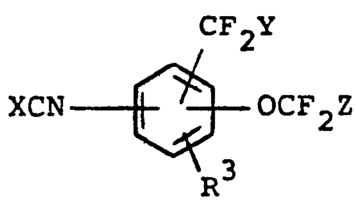

(IV)

in welcher

$R^3$, X, Y und Z die oben angegebene Bedeutung haben,

mit Benzoesäureamiden der allgemeinen Formel (V)

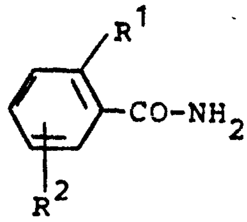

(V)

in welcher

$R^1$ und $R^2$ die oben angegebene Bedeutung haben,

gegebenenfalls in Gegenwart eines Katalysators und gegebenenfalls in Gegenwart eines Verdünnungsmittels umsetzt.

Alkyl $R^1$ und $R^2$ bedeutet geradkettiges oder verzweigtes Alkyl mit 1 bis 6, vorzugsweise 1 bis 4, insbesondere 1 oder 2 Kohlenstoffatomen. Beispielhaft seien Methyl, Ethyl, n-Propyl, i-Propyl, n-Butyl, i-Butyl, sec.-Butyl und tert.-Butyl genannt.

Halogen bedeutet Fluor, Chlor, Brom und Jod, vorzugsweise Fluor, Chlor und Brom, insbesondere Fluor oder Chlor.

Der Rest $R^2$ steht vorzugsweise in der 6-Position des Phenylringes.

In den allgemeinen Formeln (I), (II) und (IV) befinden sich im Phenylring vorzugsweise die $-CF_2Y$-Gruppe in 3-Stellung, die $-OCF_2Z$-Gruppe in 4-Stellung und $R^3$ in 2-Stellung zur -NH-, $NH_2$- oder XCN-Gruppe oder die $-CF_2Y$-Gruppe in 4-Stellung, die $-OCF_2Z$-Gruppe in 3-Stellung und $R^3$ in 2-Stellung zur -NH-, $NH_2$- oder XCN-Gruppe.

Die neuen Verbindungen der allgemeinen Formel (I) verfügen über Eigenschaften, die ihre Verwendung als Schädlingsbekämpfungsmittel ermöglichen, insbesondere zeichnen sie sich durch eine hervorragende insektizide Wirksamkeit aus.

Die Erfindung betrifft vorzugsweise neue Verbindungen der allgemeinen Formel (I), in welcher

X für Sauerstoff oder Schwefel steht,

$R^1$ für Halogen oder $C_1$-$C_6$-Alkyl steht,

$R^2$ für Wasserstoff, Halogen oder $C_1$-$C_6$-Alkyl steht und

$R^3$ für Wasserstoff, Fluor oder Chlor steht und

Y und Z gleich oder verschieden sind und für Fluor oder Chlor stehen.

Besonders bevorzugt werden Verbindungen der allgemeinen Formel (I), in welcher

X für Sauerstoff oder Schwefel steht,

$R^1$ für Fluor, Chlor, Brom oder $C_1$-$C_4$-Alkyl steht,

$R^2$ für Wasserstoff, Fluor, Chlor, Brom oder $C_1$-$C_4$-Alkyl steht und

$R^3$ für Wasserstoff, Fluor oder Chlor steht und

Y und Z gleich oder verschieden sind und für Fluor oder Chlor stehen.

Ganz besonders bevorzugt sind Verbindungen der allgemeinen Formel I, in welcher

X für Sauerstoff oder Schwefel steht,

$R^1$ für Fluor oder Chlor steht,

$R^2$ für Fluor oder Chlor in der 6-Stellung steht, und

$R^3$ für Wasserstoff steht,

Y für Fluor steht und

Z für Fluor oder Chlor steht.

Verwendet man nach Verfahrensvariante (a) 4-Trifluormethoxy-3-trifluormethyl-anilin und 2,6-Difluorbenzoylisocyanat als Ausgangsstoffe, so kann der Reaktionsablauf durch das folgende Formelschema wiedergegeben werden:

a)

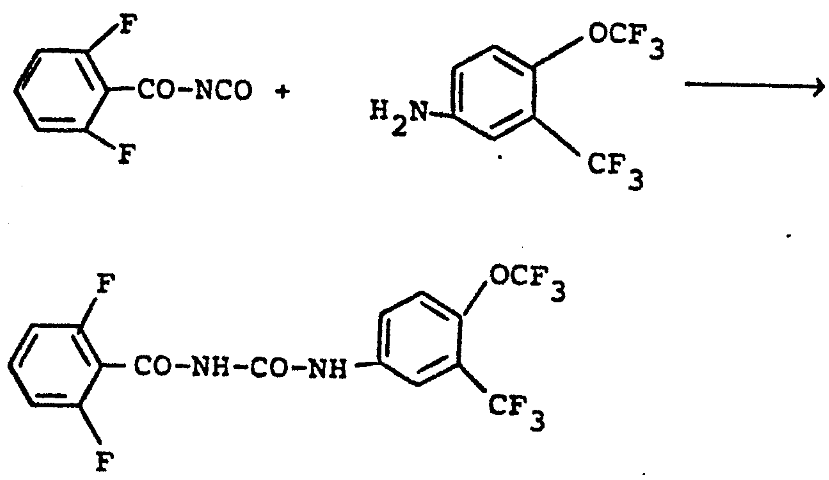

Verwendet man nach Verfahrensvariante (b) 4-Trifluormethoxy-3-trifluormethyl-isocyanat und 2,6-Difluorbenzamid als Ausgangsstoffe, so kann der Reaktionsablauf durch das folgende Formelschema wiedergegeben werden:

b) 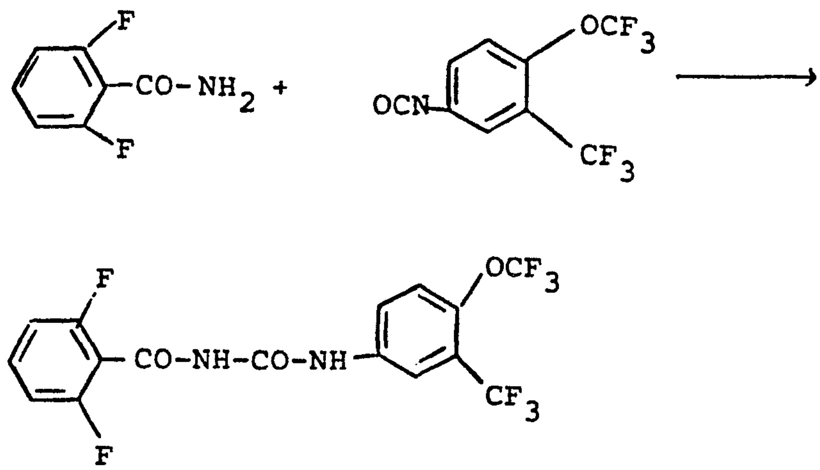

Die Ausgangsverbindungen der allgemeinen Formel (II) und (IV) sind neu. Ihre Herstellungsverfahren werden weiter unten beschrieben.

Die Ausgangsverbindungen der Formel (III) sind bekannt oder können nach allgemein bekannten Methoden erhalten werden.

Als Beispiele für die Verbindungen der allgemeinen Formel (III) seien genannt: 2-Fluor-, 2-Chlor-, 2-Brom-, 2-Methyl-, 2,6-Difluor-, 2,6-Dichlor-, 2-Chlor-6-fluor-, 2-Chlor-4-methyl, 2,6-Difluor-, 2,4-Dichlor- und 2,6-Dimethylbenzoylisocyanat bzw. -benzoylisothiocyanat.

Die Ausgangsverbindungen der allgemeinen Formel (V) sind bekannt oder können nach allgemein bekannten Methoden erhalten werden.

0180818

Als Beispiele für die Verbindungen der Formel (V) seien genannt: 2-Fluor-, 2-Chlor-, 2-Brom-, 2-Methyl-, 2,6-Difluor-, 2,6-Dichlor-, 2-Chlor-6-fluor-, 2-Chlor-4-methyl-, 2,4-Difluor-, 2,5-Dichlor- und 2,6-Dimethylbenzoesäureamid.

Als Verdünnungsmittel kommen bei den Verfahrensvarianten a) und b) praktisch alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone, wie Aceton, Methyl-ethyl-, Methylisopropyl- und Methylisobutylketon, Ester, wie Essigsäuremethylester und -ethylester, Nitrile, wie z.B. Acetonitril und Propionitril, Amide, wie z.B. Dimethylacetamid und N-Methyl-pyrrolidon, sowie Tetramethylensulfon.

Als Katalysatoren können für die Umsetzung gemäß Verfahrensvariante (b) vorzugsweise tertiäre Amine, wie Triethylamin und 1,4-Diazabicyclo/2,2,2/-octan, sowie organische Zinn-Verbindungen, wie z.B. Dibutylzinndilaurat verwendet werden. Das Verfahren kann jedoch auch ohne solche Katalysatoren durchgeführt werden.

Die Reaktionstemperatur kann bei beiden Verfahrensvarianten innerhalb eines größeren Bereiches variiert werden. Im allgemeinen arbeitet man bei der Verfahrensvariante a) zwischen 20 und 180°C, vorzugsweise zwischen 40 und 120°C und bei der Verfahrensvariante b) zwischen 20 und 200°C, vorzugsweise zwischen 60 und 190°C. Die erfindungsgemäßen Verfahrensvarianten werden im allgemeinen bei Normaldruck durchgeführt.

Zur Durchführung der erfindungsgemäßen Verfahrensvarianten werden die Ausgangsstoffe gewöhnlich in etwa äquimolaren Mengen eingesetzt. Ein Überschuß der einen oder anderen Reaktionskomponente bringt keine wesentlichen Vorteile.

Die Aufarbeitung der Reaktionsprodukte geschieht nach üblichen Methoden, z.B. durch Absaugen des ausgefallenen Produktes oder durch Herauslösen von unerwünschten Nebenprodukten aus dem Reaktionsgemisch. Zur Charakterisierung dient der Schmelzpunkt.

Die Verbindungen der allgemeinen Formeln (II) und (IV) sind neu. Sie können unter der allgemeinen Formel (VI) zusammengefaßt werden:

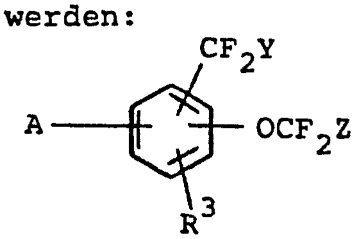

(VI)

in welcher

A für $NH_2$ oder NCX steht, wobei

X Sauerstoff oder Schwefel bedeutet, und

$R^3$ für Wasserstoff, Fluor oder Chlor steht und

Y und Z gleich oder verschieden sind und für Fluor oder Chlor stehen.

Diese Verbindungen sowie Verfahren zu ihrer Herstellung sind Teil der vorliegenden Erfindung.

Die Verbindungen der allgemeinen Formel (VI) werden dadurch erhalten, daß man Verbindungen der allgemeinen Formel (VII)

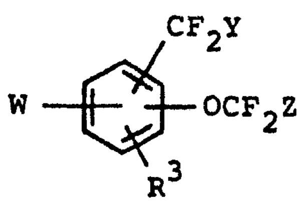

(VII)

in welcher

W für $NO_2$, COF oder CN steht,

$R^3$ für Wasserstoff, Fluor oder Chlor steht und

Y und Z gleich oder verschieden sind und für Fluor oder Chlor stehen,

für den Fall, daß

W für $NO_2$ steht,

in üblicher Weise reduziert, vorzugsweise durch katalytische Hydrierung oder mit Hilfe von Metallen oder Metallsalzen und für den Fall, daß

W für COF oder CN steht,

in üblicher Weise zu den Säureamiden hydrolysiert und die Säureamide in üblicher Weise mit Alkalihypobromit (z.B. Natriumhypobromit) zu den Aminen umsetzt (Hofmann-Abbau) und den erhaltenen Verbindungen der allgemeinen Formel (VI), wobei A für $NH_2$ steht, nach üblichen Methoden isoliert und gegebenenfalls mit Phosgen oder Thiophosgen in üblicher Weise zu den Verbindungen der allgemeinen Formel (VI), wobei A für NCX (X bedeutet Sauerstoff oder Schwefel) steht, umsetzt und diese Verbindungen nach üblichen Methoden isoliert.

Bevorzugt werden für dieses Verfahren Verbindungen der allgemeinen Formel (VII) eingesetzt, in welchen W für $NO_2$ steht.

Für die katalytische Hydrierung der Nitroverbindungen der allgemeinen Formel (VII) (W bedeutet $NO_2$) kommen alle üblichen Katalysatoren in Frage, wie Raney-Nickel, Platin, Platinoxid und Palladium, wobei die Katalysatoren auch auf Trägern, z.B. Aktivkohle oder Aluminiumoxid vorliegen können.

Als Lösungsmittel kommen alle bei Hydrierungen üblicherweise eingesetzten Lösungsmittel in Frage, wie Alkohole, z.B. Methanol oder Ether, z.B. Dioxan und Tetrahydrofuran.

Im allgemeinen setzt man erhöhte Wasserstoffdrücke, vorzugsweise zwischen 10 und 80 bar ein. Die Hydrierung wird vorzugsweise bei Temperaturen von 10 bis 100, insbesondere 20 bis 80°C durchgeführt. Die Aufarbeitung und Isolierung der Verbindungen der allgemeinen Formel (VI) (A bedeutet $NH_2$) bzw. (II) erfolgt in üblicher Weise.

Die Reduktion der Nitroverbindungen der allgemeinen Formel (VII) (W bedeutet $NO_2$) kann in üblicher Weise auch mit Metallen wie Eisen und Zinn und deren Salzen, vorzugsweise $SnCl_2$, vorgenommen werden. Die Reduktion mit $SnCl_2$ erfolgt vorzugsweise in einem Gemisch aus wäßrigen HCl und Dioxan (oder auch wasserfrei in Alkoholen, wie Ethanol) bei Temperaturen zwischen 20 und 100°C. Analog kann auch Eisen in Form von Eisenpulver oder von Eisenspänen verwendet werden, wobei vorzugsweise Temperaturen von 80 bis 100°C eingesetzt werden. Die Aufarbeitung und Isolierung der Verbindungen der allgemeinen Formel (VI) (A bedeutet $NH_2$) bzw. (II) erfolgt auch hierbei in üblicher Weise.

Die Verbindungen der allgemeinen Formel (VI) (A bedeutet NCX) bzw. (IV) können nach den üblichen Phosgenierungsmethoden leicht aus den Verbindungen der allgemeinen Formel (VI) (A bedeutet $NH_2$) bzw. (II) und Phosgen bzw. Thiophosgen erhalten werden. Als Lösungsmittel werden

vorzugsweise gegebenenfalls halogenierte aromatische oder aliphatische Lösungsmittel wie Chlortoluol, Toluol oder Xylol verwendet.

Die Phosgenierung erfolgt vorzugsweise bei Temperaturen zwischen 0 und 150, insbesondere zwischen 0 und 130°C.

Die Aufarbeitung und Isolierung der Verbindungen der allgemeinen Formel (VI) (A bedeutet NCX) bzw. (IV) wird nach allgemein üblichen Methoden vorgenommen.

Die Verbindungen der allgemeinen Formel (VII) sind neu. Diese Verbindungen sowie das Verfahren zu ihrer Herstellung sind ebenfalls Teil der vorliegenden Erfindung.

Die Verbindungen der allgemeinen Formel (VII) werden dadurch erhalten, daß man Verbindungen der allgemeinen Formel (VIII)

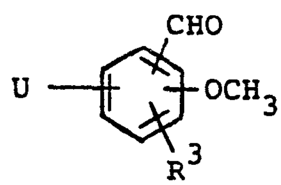

(VIII)

in welcher

U für Wasserstoff, CN oder COCl steht und

$R^3$ für Wasserstoff, Fluor oder Chlor steht,

(a) mit wenigstens der molaren Menge Phosphorpentachlorid und wenigstens der vierfach molaren Menge Chlor umsetzt und

(b) die erhaltenen Chlorierungsprodukte der allgemeinen Formel (IX)

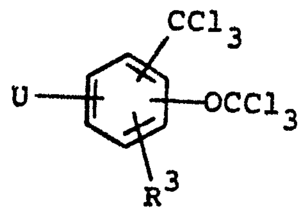

(IX)

in welcher U und $R^3$ die oben angegebene Bedeutung haben, nach üblichen Methoden isoliert und

mit wasserfreiem Fluorwasserstoff oder Antimontrifluorid, gegebenenfalls in Gegenwart eines Katalysators fluoriert, die erhaltenen Fluorierungsprodukte der allgemeinen Formel (X)

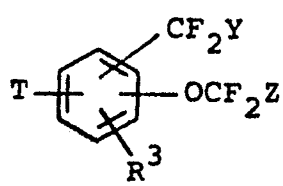

(X)

in welcher

T für Wasserstoff, CN oder COF steht,

Y und Z gleich oder verschieden sind und für Fluor oder Chlor stehen und

$R^3$ für Wasserstoff, Fluor oder Chlor steht,

nach üblichen Methoden isoliert und

(c) gegebenenfalls (zur Herstellung der Verbindungen, in welchen W für $NO_2$ steht) in üblicher Weise mit Nitriersäure nitriert und die Nitroverbindungen nach üblichen Methoden isoliert.

Gemäß diesem Verfahren ist es auch möglich, in günstiger Weise Verbindungen der allgemeinen Formel (VII) herzustellen, in welchen W die zusätzliche Bedeutung der Gruppe -OCOF hat, indem man Verbindungen der allgemeinen Formel (VIII) einsetzt, in welchen U die (zusätzliche) Bedeutung der Gruppe -OCOCl hat.

Es ist überaus überraschend, daß das erfindungsgemäße Verfahren die Verbindungen der allgemeinen Formel (VII) in guten Ausbeuten und Reinheit liefert. Es ist z.B. bekannt (Chemistry and Industry 1977, Seite 127), daß bei der Chlorierung von Methoxybenzolen mit elektronenliefernden Substituenten die Kernchlorierung zur Hauptreaktion wird. Überraschenderweise findet beim erfindungsgemäßen Verfahren eine Kernchlorierung nicht oder in vernachläßigbarem Maße statt.

Die Ausgangsverbindungen der allgemeinen Formel (VIII) sind bekannt oder können nach allgemein üblichen Methoden erhalten werden.

Im ersten Teilschritt der Chlorierungsreaktion (a) setzt man vorzugsweise 1 bis 2 Mol, insbesondere 1,0 bis 1,3 Mol Phosphorpentachlorid auf 1 Mol der Verbindung der

allgemeinen Formel (VIII) ein. Die Reaktanten werden bei Temperaturen von 0 bis 100°C, vorzugsweise 10 bis 60°C, gegebenenfalls in Gegenwart eines für Chlorierungsreaktionen üblichen Lösungsmittels, wie Phosphoroxichlorid und/oder Tetrachlormethan gemischt und zur Reaktion gebracht.

Im zweiten Teilschritt der Chlorierungsreaktion (a) setzt man vorzugsweise 3 bis 10, insbesondere 4 bis 6 Mol Chlor auf 1 Mol der Verbindung der allgemeinen Formel (VIII) ein, wobei auch ein größerer Chlorüberschuß ohne Nachteile ist. Die Umsetzung wird bei Tempraturen von 60 bis 200°C, vorzugsweise 80 bis 150°C vorgenommen. Im Temperaturbereich bis 150°C sollte die Reaktion katalytisch beschleunigt werden.

Dies kann in üblicher Weise entweder durch die Bestrahlung mit Licht (vorzugsweise UV-Licht) oder durch die Zugabe von Katalysatoren, vorzugsweise üblicher Radikalstarter, wie Dibenzoylperoxid, geschehen.

Die Chlorierungsprodukte der allgemeinen Formel (IX) werden in üblicher Weise, z.B. durch Destillation isoliert.

Die Verbindungen der allgemeinen Formel (IX) werden im Reaktionsschritt (b) mit wasserfreiem Fluorwasserstoff oder mit Antimontrifluorid fluoriert. Hierbei wird wenigstens die theoretisch benötigte Menge Fluorwasserstoff eingesetzt. Im allgemeinen wird jedoch ein HF-Überschuß verwendet. Das Antimontrifluorid wird, bezogen auf die Zahl der Chloratome, welche durch Fluor ersetzt werden sollen, in Mengen von 1,5 bis 3 Mol je Mol der

Verbindung der allgemeinen Formel (IX) eingesetzt. Die Fluorierungsreaktion kann durch die Zugabe eines üblicherweise bei Fluorierungen verwendeten Katalysators, wie Bortrifluorid, Antimonpentafluorid, Antimontrifluordichlorid, Antimonpentachlorid, Molybdänpentafluorid oder Molybdänpentachlorid, erleichtert werden. Die Gegenwart eines Katalysators ist jedoch nicht unbedingt erforderlich. Die Reaktionstemperaturen im Reaktionsschritt (b) richten sich nach dem jeweils gewünschten Fluorierungsgrad, der Menge an eingesetztem Fluorierungsmittel und Katalysator. Sie liegen zwischen 0 und 150°C, vorzugsweise zwischen 5 und 130°C. Die jeweils günstigsten Temperaturen können leicht experimentell ermittelt werden. Bei niedrigen Temperaturen zwischen 0 und 80°C werden überwiegend Verbindungen mit $CF_2Cl$- und/oder $OCF_2Cl$-Gruppen erhalten, während bei höheren Temperaturen zwischen 80 und 150°C überweigend Verbindungen mit $CF_3$- und $OCF_3$-Gruppen erzielt werden. Gegebenenfalls entstehende Produktgemische unterschiedlicher Fluorierungsgrade können leicht destillativ getrennt und isoliert werden. Die Fluorierungsstufe (b) kann unter Verwendung der bei Fluorierungsreaktionen üblichen Lösungsmittel durchgeführt werden. Vorzugsweise wird jedoch ohne die Zugabe von Lösungsmittel gearbeitet.

Die zur Herstellung der Verbindungen der allgemeinen Formel (VII), in welchen W für $NO_2$ steht, erforderliche Nitrierung der Verbindungen der allgemeinen Formel (X) erfolgt nach allgemein üblichen Methoden der Kernnitrierung. Vorzugsweise wird bei Temperaturen zwischen 10 und 50°C, insbesondere zwischen 20 und 40°C die Nitriersäure

(Mischung aus konz. Schwefelsäure und konz. Salpetersäure) ohne die Verwendung eines Lösungsmittels langsam zu der Verbindung der allgemeinen Formel (X) hinzugefügt und das Reaktionsgemisch 1 bis 3 Stunden bei dieser Temperatur gehalten. Anschließend wird das Reaktionsgemisch auf Eis gegossen, wobei sich das gewünschte Produkt als organische Phase abscheidet und leicht abtrennen läßt. Die Reinigung kann destillativ erfolgen.

Die neuen Verbindungen der allgemeinen Formeln (II), (IV) und (VII) können in der folgenden allgemeinen Formel (XI) zusammengefaßt werden:

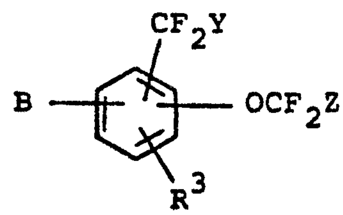

(XI)

in welcher

B für $NH_2$, $NO_2$, CN, COF oder NCX, in welcher X Sauerstoff oder Schwefel bedeutet, steht,

$R^3$ für Wasserstoff, Fluor oder Chlor steht und

Y und Z gleich oder verschieden sind und für Fluor oder Chlor stehen.

Die bevorzugten Definitionen und Stellungen der Reste in den Verbindungen der allgemeinen Formeln (VII) bis (XI) entspricht denen, welche für die Verbindungen der

allgemeinen Formeln (I) und (V) weiter oben angegeben wurden.

W in der allgemeinen Formel (VII) steht vorzugsweise für $NO_2$ und COF, insbesondere für $NO_2$.

U in den allgemeinen Formeln (VIII) und (IX) steht vorzugsweise für Wasserstoff oder COCl, insbesondere für Wasserstoff.

T in der allgemeinen Formel (X) steht vorzugsweise für Wasserstoff oder COF, insbesondere für Wasserstoff.

B in der allgemeinen Formel (XI) steht vorzugsweise für $NH_2$, $NO_2$, COF oder NCX (X = Sauerstoff oder Schwefel), insbesondere für $NH_2$, $NO_2$ oder NCX.

Die Wirkstoffe eignen sich bei guter Pflanzenverträglichkeit und günstiger Warmblütertoxizität zur Bekämpfung von tierischen Schädlingen, insbesondere Insekten, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören.

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpopha-

0180818

gus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella

aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides,

Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate;

als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäßen Wirkstoffe eignen sich auch zur Bekämpfung von unerwünschten Schädlingen auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer usw. erreicht werden können.

Die Anwendung der erfindungsgemäßen Wirkstoffe geschieht auf diesen Gebieten in bekannter Weise, wie durch äußerliche Anwendung in Form beispielsweise des Tauches (Dippen), Sprühens (Sprayen) und Aufgießens (pour-on and spot-on).

Die Herstellung der erfindungsgemäßen Verbindungen soll anhand der folgenden Beispiele erläutert werden (alle Prozentangaben beziehen sich, wo nicht anderes angegeben wird, auf Gewichtsprozente):

A Verbindungen der allgemeinen Formel (I)

Beispiel 1 A

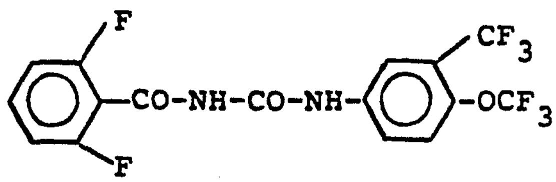

24,5 g 3-Trifluormethyl-4-trifluormethoxyanilin werden in 400 ml trockenem Toluol vorgelegt. Dazu tropft man bei 60°C eine Lösung von 18,3 g 2,6-Difluor-benzoylisocyanat in 100 ml trockenem Toluol. Der Ansatz wird eine halbe Stunde bei 80°C gerührt und anschließend etwa 2/3 des Lösungsmittels im Vakuum verdampft. Nach Zusatz von etwas Petrolether wird das ausgefallene Produkt abgetrennt. Man erhält 41 g 1-(2,6-Difluorbenzoyl)-3-(3-trifluormethyl-4-trifluormethoxy-phenyl)-harnstoff mit einem Schmelzpunkt von 178°C.

Die Umsetzung von 2,6-Difluorbenzamid mit 3-Trifluor-4-trifluormethoxy-phenyl-isocyanat in Toluol führt zum gleichen Produkt.

Analog können die in der Tabelle aufgeführten Verbindungen hergestellt werden:

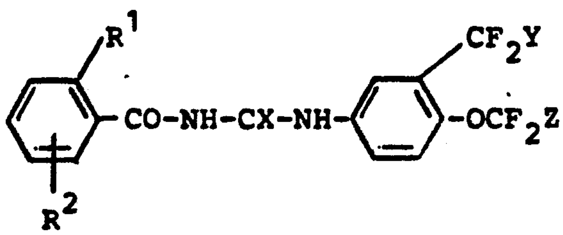

(XII)

| Beispiel-Nr. | $R_1$ | $R_2$ | Y | Z | X | Schmp. (°C) |
|---|---|---|---|---|---|---|
| 2A | Cl | 4-F | F | F | O | 167-168 |
| 3A | Cl | 5-F | F | F | O | 139 |
| 4A | Cl | 6-F | F | F | O | 166 |
| 5A | Cl | 6-F | F | F | S | 192 |
| 6A | F | 6-F | F | F | S | 178 |
| 7A | Cl | 4-F | F | F | S | 128 |
| 8A | Cl | 6-Cl | F | F | S | 211 |
| 9A | Br | H | F | F | S | 157 |
| 10A | F | 6-F | F | Cl | O | 152 |
| 11A | Cl | 4-F | F | Cl | O | 134 |
| 12A | Cl | 5-F | F | Cl | O | 120 |
| 13A | Cl | 6-F | F | Cl | S | 173 |
| 14A | F | 6-F | F | Cl | S | 156 |
| 15A | Br | H | F | Cl | S | 143 |

B Verbindungen der allgemeinen Formel (IX)

Beispiel 1B

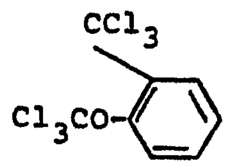

In 4000 ml Tetrachlormethan werden 1840 g Phosphorpentachlorid suspendiert und 1080 g 2-Methoxybenzaldehyd zugetropft. Nach Ende der Zugabe wird noch 2 Stunden bei 20-30°C gerührt. Dann auf 80°C (Rückfluß) erhitzt und unter UV-Bestrahlung chloriert (ca. 2 kg Chlor eingeleitet).

Durch Destillation der Reaktionsmischung werden 2015 g 2-Trichlormethoxy-trichlormethylbenzol, Kp: 120-24°C/ 0,2 mbar, $n_D^{20}$: 1,5765 erhalten (Reinheit 98,5 %).

Der Vorlauf an unterchlorierten Verbindungen kann erneut zur Chlorierung eingesetzt werden. Die Chlorierung erfolgt dann ohne Lösungsmittel bei ca. 110-140°C.

Beispiel 2B

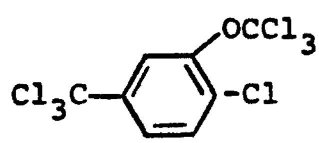

In einer Rührapparatur werden 1200 ml Tetrachlormethan und 200 g Phosphorpentachlorid bei 20°C vorgelegt und 160 g 4-Chlor-3-methoxy-benzaldehyd zugetropft. Man rührt 1 Stunde bei 20-30°C und 1 Stunde bei 50°C, überführt die Reaktionsmischung in eine Chlorierungsapparatur mit

einer Tauchlampe und erhitzt auf 80°C unter UV-Bestrahlung. Dann wird für 8 Stunden Chlor durchgeleitet. Nach gaschromatographischer Analyse beträgt der Anteil des gewünschten Produktes 85 %. Durch Destillation wird das 4-Chlor-3-trichlormethoxy-trichlormethylbenzol isoliert (Kp: 170-6°C/1 mbar, $n_D^{20}$: 1.5760, Ausbeute: 246 g).

Analog werden folgende Verbindungen erhalten:

| Beispiel-Nr. | | | |
|---|---|---|---|
| 3B | 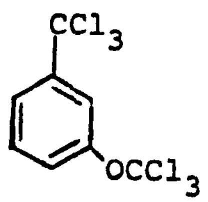 | Kp: 128-33°C/0,6 mbar | $n_D^{20}$: 1.5735 |
| 4B | 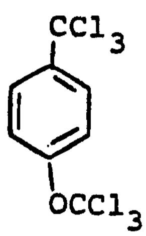 | Kp: 130-5°C/0,8 mbar | $n_D^{20}$: 1.5742 |
| 5B | 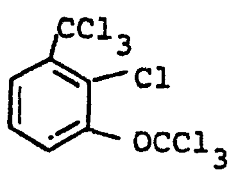 | Kp: 143-48°C/0,1 mbar | - |
| 6B | 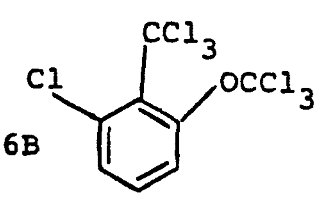 | Kp: 132-8°C/0,05 mbar | $n_D^{20}$: 1.5878 |

Beispiel-
Nr.

7B 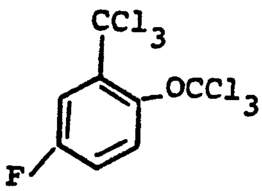 Kp: 115-18°C/0,35 mbar $n_D^{20}$: 1.5556

8B 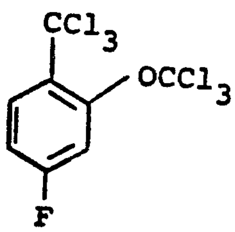 Kp: 112-5°C/0,2 mbar $n_D^{20}$: 1.5545

9B 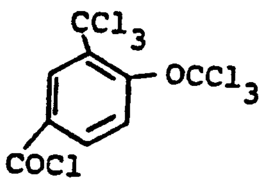 Kp: 154-60°C/0,02 mbar $n_D^{20}$: 1,.5910

C Verbindungen der allgemeinen Formel (X)

Beispiel 1C

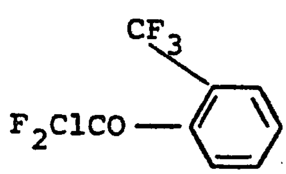

In einer Fluorierungsapparatur aus Edelstahl (V4A-Stahl) werden 240 ml wasserfreier Fluorwasserstoff bei ca. 0°C vorgelegt und 220 g 2-Trichlormethoxy-trichlormethylbenzol zugetropft. Anschließend wird erwärmt und bei 8-10°C setzt zügige HCl-Entwicklung ein. Nachdem die Chlorwasserstoff-Entwicklung bei 20°C beendet ist, werden ca. 3 bar Stickstoff aufgedrückt und die Temperatur auf 60°C, nach einer Stunde auf 100°C für 3 Stunden erhöht. Dabei wird über einen Rückflußkühler mit Entspannungsventil der entstehende Chlorwasserstoff abgeblasen. Nach Abkühlen auf ca. 20°C wird der überschüssige Fluorwasserstoff abdestilliert und das Reaktionsprodukt über eine Kolonne destillativ gereinigt (Kp: 163-5°C, Kp: 55-58°C/14 mbar, $n_D^{20}$: 1.4180). Die Ausbeute an 2-Difluorchlormethoxy-trifluormethylbenzol beträgt 119 g.

Beispiel 2C

In der vorher beschriebenen Fluorierungsapparatur werden 120 ml Fluorwasserstoff und 110 g 2-Trichlormethoxy-trichlormethylbenzol bei 5 bis 20°C für 8 Stunden zur Reaktion gebracht.

Durch Destillation über eine Drehbandkolonne werden folgende Verbindungen isoliert:

2-Difluorchlormethoxy-difluorchlormethylbenzol

Kp: 94-5°C/18 mbar $n_D^{20}$: 1.4685

2-Fluordichlormethoxy-difluorchlormethylbenzol

Kp: 102-4°C/18 mbar $n_D^{20}$: 1.4838

(neben 2-Difluorchlormethoxy-trifluormethylbenzol)

Beispiel 3C

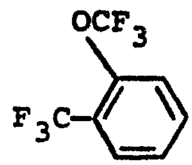

Zu 500 g 2-Difluorchlormethoxy-trifluormethylbenzol gibt man 200 g sublimiertes Antimontrifluorid und 5 ml Antimonpentachlorid, worauf eine exotherme Reaktion einsetzt. Man hält die Temperatur für 4 Stunden bei 110-120°C und destilliert anschließend das Reaktionsprodukt über. Man erhält 345 g 2-Trifluormethoxy-trifluormethylbenzol. Kp: 133-4°C; $n_D^{20}$: 1.3825.

Beispiel 4C

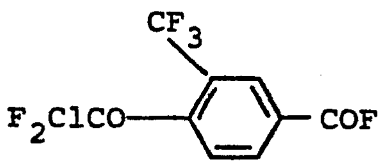

In obengenannter Apparatur werden 150 ml Fluorwasserstoff und 100 g 4-Trichlormethoxy-3-trichlormethyl-benzoylchlorid für 2 Stunden bei 60°C, 2 Stunden bei 100°C und 3 Stunden bei 120°C zur Reaktion gebracht.

Durch Destillation werden 55 g 4-Difluorchlormethoxy-3-trifluormethyl-benzoylfluorid (Kp: 85-7°C/18 mbar, $n_D^{20}$: 1.4340) erhalten.

Analog den Beispielen 1C bis 4C werden folgende Verbindungen erhalten:

0180818

| Beispiel-Nr. | | | |
|---|---|---|---|
| 5C | 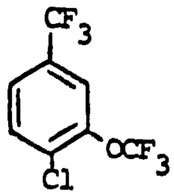 | Kp: 43-5°C/18 mbar | $n_D^{20}$: 1.4035 |
| 6C | 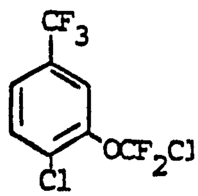 | Kp: 67-8°C/18 mbar | $n_D^{20}$: 1.4329 |
| 7C | 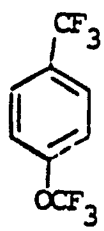 | Kp: 131-3°C | $n_D^{20}$: 1,3815 |
| 8C | 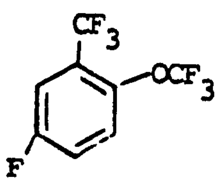 | Kp: 124-7°C | $n_D^{20}$: 1,3720 |
| 9C | 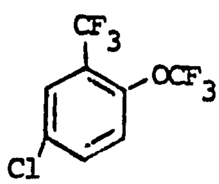 | Kp: 152-5°C | $n_D^{20}$: 1.4040 |
| 10C | 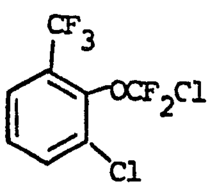 | Kp: 176-9°C | $n_D^{20}$: 1.4352 |

## D Verbindungen der allgemeinen Formel (VII) mit W = $NO_2$

Beispiel 1D

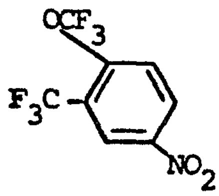

In einer Rührapparatur werden 54 g 2-Trifluormethoxy-trifluormethylbenzol bei 30-35°C vorgelegt und 54 g Mischsäure (Nitriersäure) zugetropft. Die Reaktion ist exotherm, man hält für 2 Stunden bei 30°C, gießt dann auf Eis und trennt das 2-Trifluormethoxy-5-nitro-trifluormethylbenzol ab (Kp: 87-90°C/18 mbar, $n_D^{20}$: 1.4315). Ausbeute: 56 g

Analog werden erhalten:

| Beispiel-Nr. | | | |
|---|---|---|---|
| 2D | 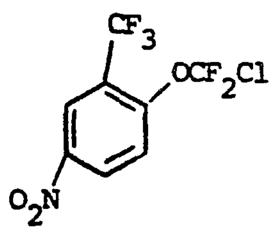 | Kp: 107-8°C/16 mbar | $n_D^{20}$: 1.4595 |
| * 3D | 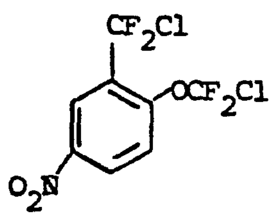 | Kp: 120-5°C/12 mbar | $n_D^{20}$: 1.4880 |

| Beispiel-Nr. | | | |
|---|---|---|---|
| 4D | 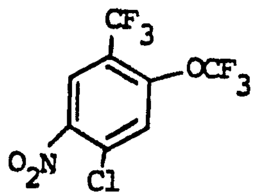 | Kp: 102-6°C/18 mbar | $n_D^{20}$: 1.4508 |
| 5D | 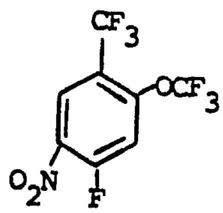 | Kp: 78-80°C/10 mbar | $n_D^{20}$: 1.4160 |
| 6D | 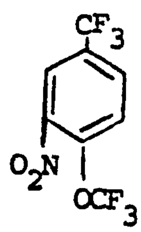 | Kp: 83-6°C/14 mbar | $n_D^{20}$: 1.4312 |

* unter Zusatz von 20 %igem Oleum bei 80-85°C nitriert.

## E Verbindungen der allgemeinen Formel (II)

### Beispiel 1E

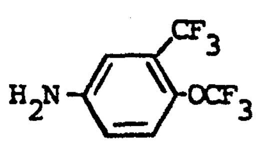

In einer Hydrierapparatur werden 317 g 3-Trifluormethoxy-5-nitro-trifluormethylbenzol in 1200 ml Methanol und 30 g Raney-Nickel vorgelegt und mit einem Wasserstoffdruck von 30-50 bar bei 20-45°C hydriert.

Nach Destillation werden 222 g 4-Trifluormethoxy-3-trifluormethyl-anilin (Kp: 90-92°C/16 mbar, $n_D^{20}$: 1.4295) erhalten.

<u>Beispiel 2E</u>

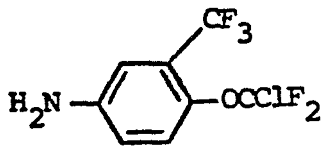

Man legt 150 g Zinnchlorid-dihydrat in 160 ml konz. Salzsäure bei 50°C vor und tropft 85 g 2-Difluorchlormethoxy-5-nitro-trifluormethylbenzol gelöst in 100 ml Dioxan zu. Nach Ende der Zugabe wird noch für 2 Stunden unter Rückfluß erhitzt, abgekühlt, mit 300 ml Wasser verdünnt und durch Eintropfen von 40 %iger Natronlauge alkalisch gestellt. Nach Extrahieren mit Dichlormethan und Trocknen der organischen Phase wird das Lösungsmittel im Vakuum abgezogen und das Rohprodukt destilliert. Man erhält 62 g 4-Difluorchlormethoxy-3-trifluormethyl-anilin (Kp: 110-113°C/16 mbar, $n_D^{20}$ : 1,4615).

Analog den vorangegangenen Beispielen 1E und 2E werden erhalten:

| Beispiel-Nr. | | | |
|---|---|---|---|
| 3E | 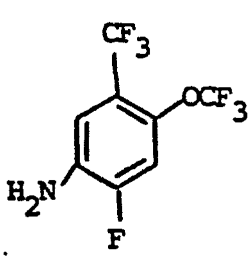 | Kp: 85-87°C/14 mbar | $n_D^{20}$ : 1,4085 |

Beispiel-
Nr.

4E 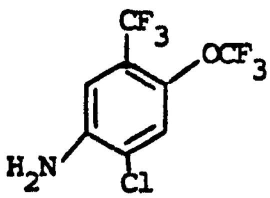 Kp: 140-4°C/20 mbar

5E 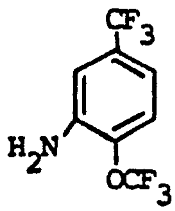 Kp: 74-8°C/18 mbar $n_D^{20}$: 1.4090

F Verbindungen der allgemeinen Formel (IV)

Beispiel 1F

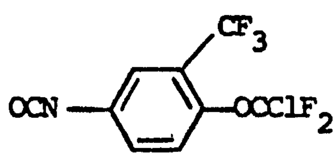

In 400 ml Chlorbenzol werden 40 g 4-Difluorchlormethoxy-3-trifluormethyl-anilin bei 0°C vorgelegt, ca. 40 g Phosgen eingeleitet und anschließend die Temperatur während der Phosgenierung bis max. 130°C gesteigert. Nach Ausblasen mit Stickstoff wird destilliert.

Man erhält 40 g 4-Difluorchlormethoxy-3-trifluormethyl-phenylisocyanat (Kp: 93-5°C/16 mbar, $n_D^{20}$: 1.4580).

Beispiel 2F

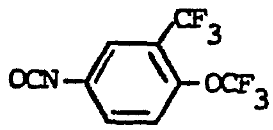

Analog der vorher beschriebenen Verfahrensweise wird das 4-Trifluormethoxy-3-trifluormethyl-phenylisocyanat erhalten (Kp: 70-2°C/14 mbar).

Die biologische Wirksamkeit der erfindungsgemäßen Verbindungen der Formel (I) soll anhand der folgenden Beispiele erläutert werden:

Beispiel A

Phaedon-Larven-Test

Lösungsmittel: 15 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käferlarven abgetötet wurden; 0 % bedeutet, daß keine Käfer-Larven abgetötet wurden.

Bei diesem Test zeigten beispielsweise die Verbindungen der Herstellungsbeispiele 1A, 4A, 6A und 10A bei einer Wirkstoffkonzentration von 0,01% nach 10 Tagen eine Abtötung von 100 %.

**Beispiel B**

Plutella-Test

Lösungsmittel: 15 Gewichtsteile Dimethylformamid
Emulgator : 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigten beispielsweise die Verbindungen der Herstellungsbeispiele 1A, 2A, 4A und 10A bei einer Wirkstoffkonzentration von 0,0001 % nach 7 Tagen eine Abtötung von 100 %.

Beispiel C

Test mit Lucilia cuprina resistent-Larven

Emulgator: 35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Gemisches und verdünnnt das so erhaltene Konzentrat mit Wasser auf die jeweils gewünschte Konzentration.

Etwa 20 Lucilia cuprina res.-Larven werden in ein Teströhrchen gebracht, welches ca. 1 $cm^3$ Pferdefleisch und 0,5 ml der Wirkstoffzubereitung enthält. Nach 24 Stunden wird der Abtötungsgrad bestimmt.

Bei diesem Test zeigte beispielsweise die Verbindung des Herstellungsbeispiels 1A bei einer Wirkstoffkonzentration von 300 ppm eine 100 %ige Wirkung.

Patentansprüche

1. Verbindungen der allgemeinen Formel (I)

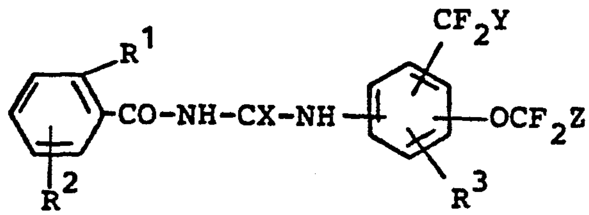

(I)

in welcher

$R^1$ für Halogen oder Alkyl steht,

$R^2$ für Wasserstoff, Halogen oder Alkyl,

$R^3$ für Wasserstoff, Fluor oder Chlor steht,

X für Sauerstoff oder Schwefel steht, und

Y und Z gleich oder verschieden sind und für Fluor oder Chlor stehen.

2. Verbindungen der allgemeinen Formel (I) gemäß Anspruch 1, in welcher

X für Sauerstoff oder Schwefel steht,

$R^1$ für Halogen oder $C_1$-$C_6$-Alkyl steht,

$R^2$ für Wasserstoff, Halogen oder $C_1$-$C_6$-Alkyl steht und

$R^3$ für Wasserstoff, Fluor oder Chlor steht und

Y und Z gleich oder verschieden sind und für Fluor oder Chlor stehen.

3. Verfahren zur Herstellung von Verbindungen der allgemeinen Formel (I)

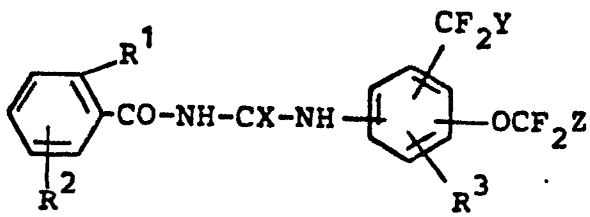

(I)

in welcher

$R^1$ für Halogen oder Alkyl steht,

$R^2$ für Wasserstoff, Halogen oder Alkyl,

$R^3$ für Wasserstoff, Fluor oder Chlor steht,

X für Sauerstoff oder Schwefel steht und

Y und Z gleich oder verschieden sind und für Fluor oder Chlor stehen,

dadurch gekennzeichnet, daß man

a) substituierte Aniline der allgemeinen Formel (II)

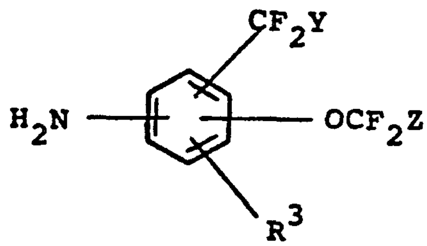

(II)

in welcher $R^3$, Y und Z die oben angebene Bedeutung haben,

mit Benzoyl-iso(thio)cyanaten der allgemeinen Formel (III)

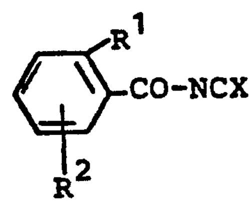

(III)

in welcher

X, $R^1$ und $R^2$ die obenangegebene Bedeutung haben,

gegebenenfalls in Gegenwart eines Verdünnungsmittels umsetzt oder

b) substituierte Phenyl-iso(thio)cyanate der allgemeinen Formel (IV)

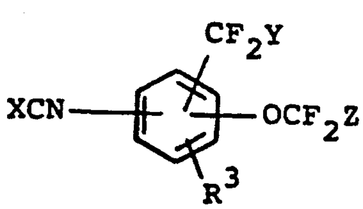

(IV)

in welcher

$R^3$, X, Y und Z die oben angegebenen Bedeutungen haben,

mit Benzoesäureamiden der allgemeinen Formel (V)

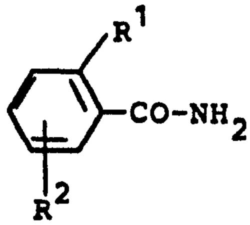

(V)

in welcher

$R^1$ und $R^2$ die oben angegebene Bedeutung haben,

gegebenenfalls in Gegenwart eines Katalysators und gegebenenfalls in Gegenwart eines Verdünnungsmittels umsetzt.

4. Schädlingsbekämpfungsmittel, gekennzeichnet durch einen Gehalt an mindestens einer Verbindung der Formel I gemäß den Ansprüchen 1 oder 3.

5. Verwendung von Verbindungen der Formel I gemäß den Ansprüchen 1 oder 3 zur Bekämpfung von Schädlingen, insbesondere Insekten.

6. Verfahren zur Bekämpfung von Schädlingen, dadurch gekennzeichnet, daß man Verbindungen Der Formel I gemäß den Ansprüchen 1 oder 3 auf die Schädlinge,

vorzugsweise Insekten oder ihren Lebensraum einwirken läßt.

7. Verbindungen der allgemeinen Formel (XI)

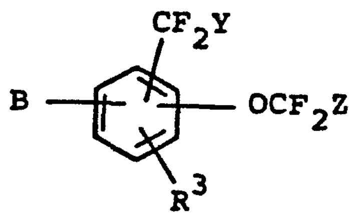

(XI)

in welcher

B für $NH_2$, $NO_2$, CN, COF oder NCX, in welcher

X Sauerstoff oder Schwefel bedeutet, steht,

$R^3$ für Wasserstoff, Fluor oder Chlor steht und

Y und Z gleich oder verschieden sind und für Fluor oder Chlor stehen.

8. Verbindungen der allgemeinen Formel (VI)

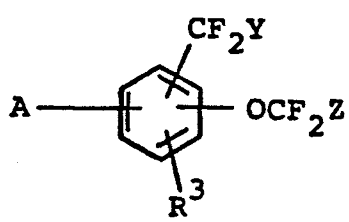

(VI)

in welcher

A für $NH_2$ oder NCX steht, wobei

X Sauerstoff oder Schwefel bedeutet, und

$R^3$ für Sauerstoff, Fluor oder Chlor steht und

Y und Z gleich oder verschieden sind und für Fluor oder Chlor stehen.

9. Verfahren zur Herstellung der Verbindungen der allgemeinen Formel (VI)

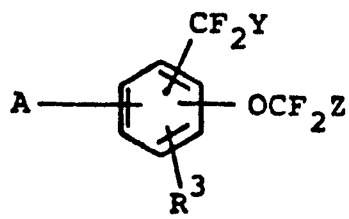

(VI)

in welcher

A für $NH_2$ oder NCX steht, wobei

X Sauerstoff oder Schwefel bedeutet, und

$R^3$ für Wasserstoff, Fluor oder Chlor steht und

Y und Z gleich oder verschieden sind und für Fluor oder Chlor stehen,

dadurch gekennzeichnet, daß man die Verbindungen der allgemeinen Formel (VII)

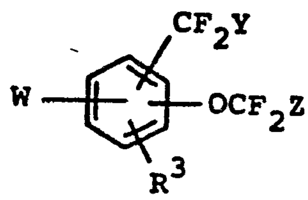

(VII)

in welcher

W für $NO_2$, COF oder CN steht,

$R^3$ für Wasserstoff, Fluor oder Chlor steht und

Y und Z gleich oder verschieden sind und für Fluor oder Chlor stehen,

für den Fall, daß

W für $NO_2$ steht,

in üblicher Weise reduziert, vorzugsweise durch katalytische Hydrierung oder mit Hilfe von Metallen oder Metallsalzen und für den Fall, daß

W für COF oder CN steht,

in üblicher Weise in die Säureamide überführt und diese Säureamide in üblicher Weise mit Alkalihypohalogenit (z.B. Natriumhypobromit) zu den Aminen umsetzt (Hofmann-Abbau) und die erhaltenen Verbindungen der allgemeinen Formel (VI), wobei A für $NH_2$ steht, nach üblichen Methoden isoliert und gegebenenfalls mit Phosgen oder Thiophosgen in üblicher

Weise zu den Verbindungen der allgemeinen Formel (VI), wobei A für NCX (X bedeutet Sauerstoff oder Schwefel) steht, umsetzt und diese Verbindungen nach üblichen Methoden isoliert.

10. Verbindungen der allgemeinen Formel (VII)

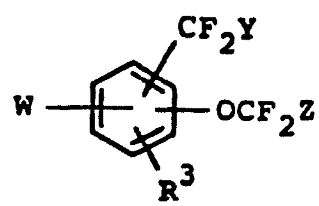

(VII)

in welcher

W für $NO_2$, COF oder CN steht,

$R^3$ für Wasserstoff, Fluor oder Chlor steht und

Y und Z gleich oder verschieden sind und für Fluor oder Chlor stehen.

11. Verfahren zur Herstellung der Verbindungen der allgemeinen Formel (VII)

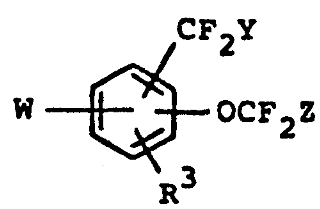

(VII)

in welcher

W für $NO_2$, COF oder CN steht,

$R^3$ für Wasserstoff, Fluor oder Chlor steht und

Y und Z gleich oder verschieden sind und für Fluor oder Chlor stehen,

dadurch gekennzeichnet, daß man Verbindungen der allgemeinen Formel (VIII)

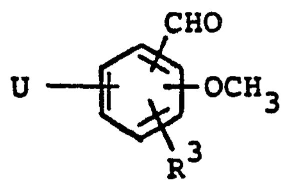

(VIII)

in welcher

U für Wasserstoff, CN oder COCl steht und

$R^3$ für Wasserstoff, Fluor oder Chlor steht,

(a) mit wenigstens den molaren Mengen Phosphorpentachlorid und wenigstens der vierfach molaren Menge Chlor umsetzt und

(b) die erhaltenen Chlorierungsprodukte der allgemeinen Formel (IX)

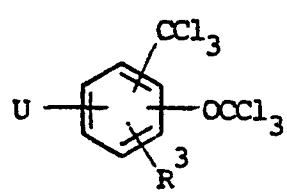

(IX)

in welcher U und $R^3$ die oben angegebene Bedeutung haben nach üblichen Methoden isoliert und mit wasserfreiem Fluorwasserstoff oder Antimontrifluorid, gegebenenfalls in Gegenwart eines Katalysators fluoriert, die erhaltenen Fluorierungsprodukte der allgemeinen Formel (X)

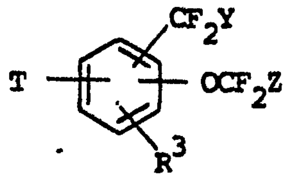 (X)

in welcher

T für Wasserstoff, CN oder COF steht,

Y und Z gleich oder verschieden sind und für Fluor oder Chlor stehen und

$R^3$ für Wasserstoff, Fluor oder Chlor steht,

nach üblichen Methoden isoliert und

(c) gegebenenfalls (zur Herstellung der Verbindungen, in welchen W für $NO_2$ steht) in üblicher Weise mit Nitriersäure nitriert und die Nitroverbindungen nach üblichen Methoden isoliert.